# EUROPEAN PATENT APPLICATION

(11) **EP 3 461 520 A1**
(43) Date of publication of application: **03.04.2019**
(21) Application number: 17194466.3
(22) Date of filing: 02.10.2017
(51) Int. Cl.: A61M 5/32, A61B 50/36

(54) **SMART CABINET FOR A DISPOSABLE CONTAINER SYSTEM**

(71) Applicant: Ares Trading S.A., 1170 Aubonne (CH)
(72) Inventor: LE MASNE, Quentin, 01220 Divonne les Bains (FR); CHATTON, Rodrigue, 1028 Préverenges (CH); PFISTER, Matthias, 3097 Liebefeld (CH); THEVENAZ, Nicolas, 1762 Givisiez (CH); PELLATON, Yves, 3232 Ins (CH)
(74) Representative: Merck Serono S.A. Intellectual Property

(57) **Abstract**

Systems disclosed herein provide for a disposal cabinet for discarding and identifying a variety of injection devices. Embodiments provide for the identification of the injection device based on at least one color and shape of the injection device. The identification of the injection device is performed with a plurality of decision trees. The injection devices are safely and efficiently discarded into a disposal container located within the disposal cabinet.

## Description

### FIELD OF THE INVENTION

The present invention relates to a disposal cabinet for safely and efficiently discarding a variety of injection devices as well as identifying the injection devices as they're being discarded.

### BACKGROUND DISCUSSION

Many people diagnosed with one or more chronic diseases are prescribed regular injections with a variety of injection devices (e.g., syringes, needles, cartridges, and auto-injectors). For those people with at least one of: dexterity impairments (muscle weakness and numbness, pain, swelling in joints, spasms), visual impairments, and cognitive impairments (memory, confusion and forgetfulness, fatigue, depression and anxiety), adhering to a prescribed treatment of regular injection may prove quite difficult. Specifically, people with the aforementioned impairments may find it difficult to properly and safely discard of the different injection. Further, such people may also find it difficult to keep track of all the different injections prescribed in the treatment. Further, unless a medical professional or caregiver is actually watching the patient inject and discard of the injection device, there is no way to verify if the patient is adhering to the prescribed treatment. Accordingly, what is need is a disposal cabinet for safely and efficiently discarding a variety of injection devices as well identifying the injection devices as they're being discarded

### BRIEF DESCRIPTION OF FIGURES

Figure 1 illustrates an example embodiment of a casing of the injection device disposal cabinet.
Figure 2A illustrates an example embodiment of an optical module of the injection device disposal cabinet.
Figure 2B illustrates an exploded view of an example embodiment of an optical module of the injection device disposal cabinet.
Figure 2C illustrates an example embodiment of a variety reflected image paths in the optical module depicted in Figure 2B.
Figure 3 illustrates an example embodiment of a transfer module of the injection device disposal cabinet.
Figure 4 illustrates an example embodiment of a control module of the injection device disposal cabinet.
Figure 5 illustrates an example embodiment of the electronics architecture of the injection device disposal cabinet.
Figure 6A illustrates an example embodiment of the process utilized to identify an injection device.
Figure 6B illustrates an example of the decision trees concept utilized in Figure 6A.
Figure 7 provides a non-exhaustive list of injection devices recognizable by the injection device disposal cabinet.
Figure 8 illustrates a flow diagram of the injection device identification process.
Figure 9 illustrates an example embodiment of a user interaction with disposal cabinet.

### DESCRIPTION OF EMBODIMENTS

The following description of embodiments provides non-limiting representative examples referencing numerals to particularly describe features and teachings of different aspects of the invention. The embodiments described should be recognized as capable of implementation separately, or in combination, with other embodiments from the description of the embodiments. A person of ordinary skill in the art reviewing the description of embodiments should be able to learn and understand the different described aspects of the invention. The description of embodiments should facilitate understanding of the invention to such an extent that other implementations, not specifically covered but within the knowledge of a person of skill in the art having read the description of embodiments, would be understood to be consistent with an application of the invention.

One aspect of the present disclosure is to provide systems for efficiently disposing and identifying a variety of different injection devices. The systems herein address at least one of the problems discussed above.

According to an embodiment, a disposal cabinet for discarding and identifying a variety of injection devices includes an optical module, wherein the optical module includes an image acquisition device; and a processor, wherein, upon detecting an object approaching the disposal cabinet, the processor is configured to: receive, in the optical module, an injection device, capture, with the image acquisition device, an image of the injection device, discard the received injection device into a disposal container coupled to the optical module, and identify the injection device based on the captured image.

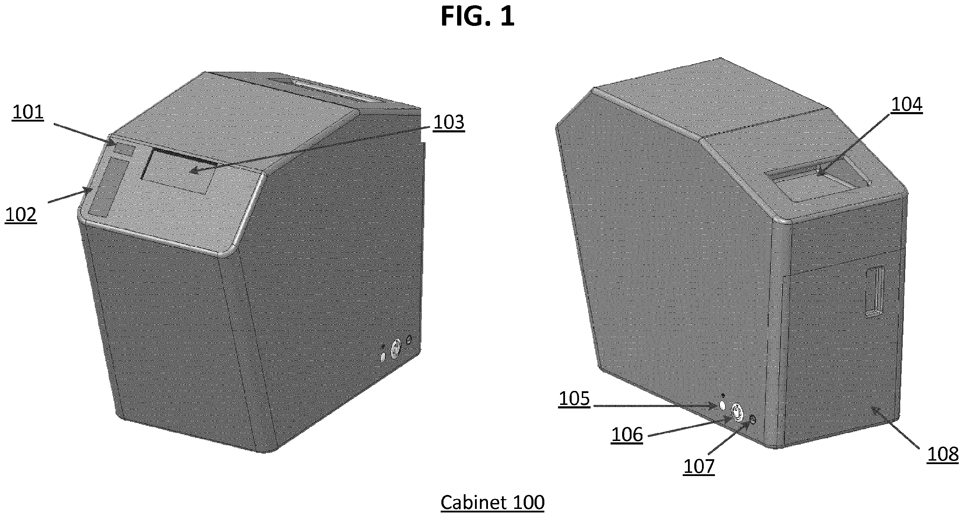

Figure 1 illustrates an example embodiment of a casing of the injection device disposal cabinet. In an embodiment, as depicted in the figure, the casing of the injection device disposal cabinet 100 includes an activity area 101, a graphical user interface 102, an opening 103, a handle 104, a pairing button 105, a power switch 106, a power plug 107, and a door 108. In an embodiment, the activity area 101 includes a proximity sensor to detect approaching objects (e.g., person's hand and/or the injection device). In an embodiment, once the proximity sensor associated with the activity area 101 detects an object approaching the disposal cabinet, the system on the disposal cabinet is activated and the opening 103 (e.g., a trapdoor) is opened in order to receive the injection device. Thus, the injection device can be disposed of with only a single hand. In an embodiment, the shape of the opening 103 is compatible to receive various injection device sizes. Therefore, users suffering from many of the impairments discussed above will be able to easily interact with the opening 103. In an embodiment, the opening 103 also shields the optical module from environmental perturbations like direct sun illumination or dust. Further, in an embodiment, the opening 103 is located near the graphical user interface 102. In an embodiment, the graphical user interface 102 informs the user about the various disposal cabinet-related states.

Further, in an embodiment, as depicted in the figure, the handle 104, the pairing button 105, the power switch 106, the power plug 107, and the door 108 can be located on a variety of different sides of the casing of the disposal cabinet 100. In an embodiment, the handle 104 facilitates the user's manipulation of the disposal cabinet 100. Therefore, the disposal cabinet 100 can be easily moved and transported to a variety of locations. Further, in an embodiment, the pairing button 105 allows the disposal cabinet 100 to pair (e.g., via a Bluetooth interface) to a user's smart device (e.g., smartphone, tablet, smart watch). Therefore, the device is able to transmit important information from the disposal cabinet 100 to the user's smart device. In an embodiment, the power switch 106 is used to activate and de-activate the power being supplied to the electrical components of the disposal cabinet 100 via an internal battery. Further, the power plug 107 is utilized to charge the internal battery as needed. Further, in an embodiment, the door 108 allows the user to place a disposal container (not shown) within the cabinet when necessary. In another embodiment, the door 108 may be implemented with a child-safety lock.

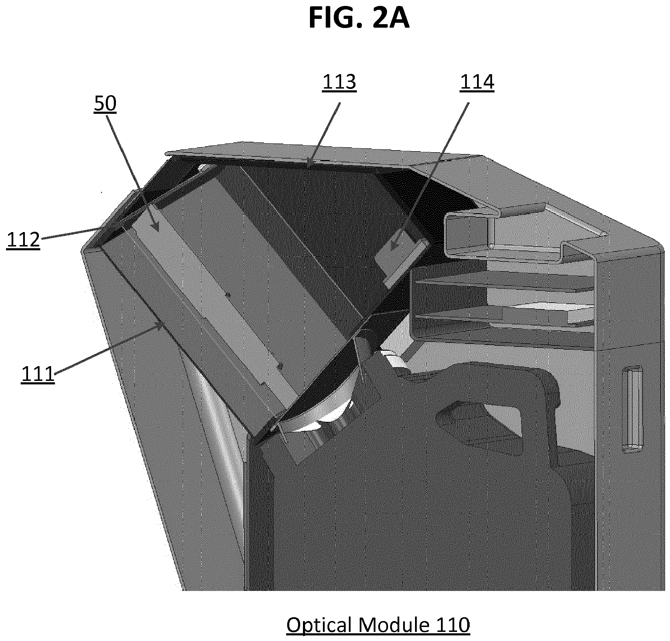

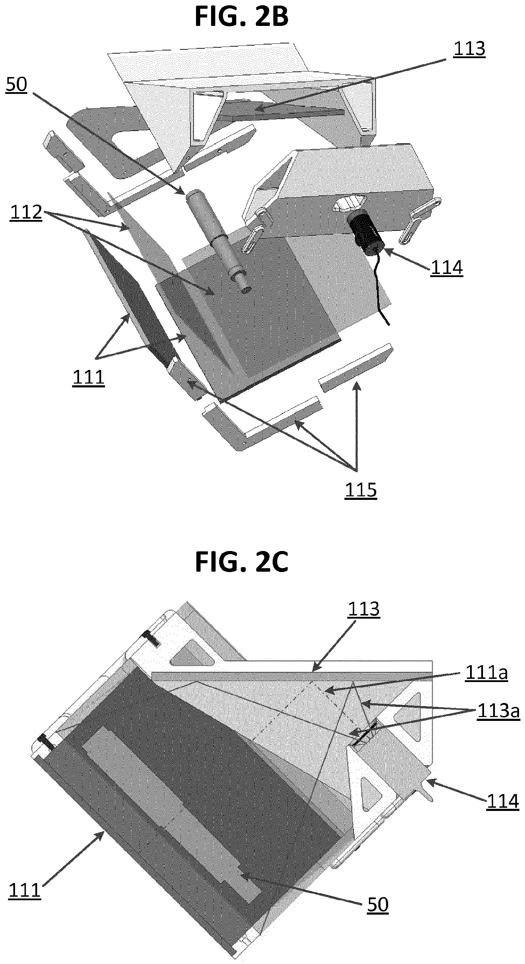

Figure 2A illustrates an example embodiment of an optical module of the injection device disposal cabinet. In an embodiment, as depicted in the figure, the optical module 110 is integrated within the disposal cabinet 100. Further, in an embodiment, the optical module 110 includes a plurality of reflecting surfaces 111, a plurality of transparent holding elements 112, a reflecting surface 113, and an image acquisition device 114. In an embodiment, the reflecting surfaces 111 are configured in a V-shape and are utilized to reflect an image of a side of an injection device 50 facing the reflecting surfaces 113 (e.g., the back side of the injection device 50). In an embodiment, the reflecting surfaces 113 are a plurality of mirrors. Further, in an embodiment, the transparent holding elements 112 also configured in a V-shape and are each located at a predefined distance from a respective reflecting surfaces 113. In an embodiment, the transparent holding elements are configured to hold the injection device 50 after it is received by the opening 103 in Figure 1. In an embodiment, the transparent holding elements 112 are a plurality of transparent glass slabs. Further, in an embodiment, the reflecting surface 113 is located at the top of the optical module 110 and is configured to reflect images of the injection device 50 to the image acquisition device 114. For example, the reflecting surface 113 is configured to reflect images of the injection device 50 initially reflected by the reflecting surfaces 111. Therefore, by the folding the optical path of the reflected images of the of the injection device 50 initially reflected by the reflecting surfaces 111, the height of the optical module 110 can be optimally minimized. In an embodiment, the image acquisition device 114 is configured to acquire an image of the injection device 50 based on the reflections from the reflecting surfaces 111 and 113. In an embodiment, the image acquisition device 114 is a CMOS image sensor configured to receive and capture color images. In another embodiment, the image acquisition device 114 is a CCD image sensor configured to receive and capture color images. In an embodiment, the captured image may then be stored for further processing (e.g., identification of the injection device 50). Figure 2B illustrates an exploded view of an example embodiment of an optical module of the injection device disposal cabinet. As depicted in the figure, the transparent holding elements 112 are maintained in the V-shape by holding elements 115 coupled to each of the transparent holding elements 112.

Figure 2C illustrates an example embodiment of a variety of reflected image paths in the optical module depicted in Figure 2B. For example, as depicted in the figure, the image acquisition device 114 receives image paths 111a and 113a. In an embodiment, the reflected image path 111a corresponds to the optical path of the reflected images of the of the injection device 50 initially reflected by the reflecting surfaces 111 and then reflected by the reflecting surface 113. Further, the reflected image paths 113a correspond to the optical paths of reflected images of the injection device 50 that were not initially reflected by the reflecting surfaces 111. In other words, the reflect image paths 113a correspond to images of the injection device 50 that were reflected only by the reflecting surface 113.

Therefore, with the image acquisition device 114 and the reflecting surface 111 and 113, an image of the injection device 50 may be captured from multiple points of view. Accordingly, a sufficient amount of information may be collected in order to determine the discriminators, such as color and shape, of the injection device 50. However, in an embodiment, additional sensing systems can also be included in order to determine other discriminators, such as weight, metallic or ferromagnetic content, Optical Character Recognition (OCR) and barcode (e.g., one-dimensional and two-dimensional) reading.

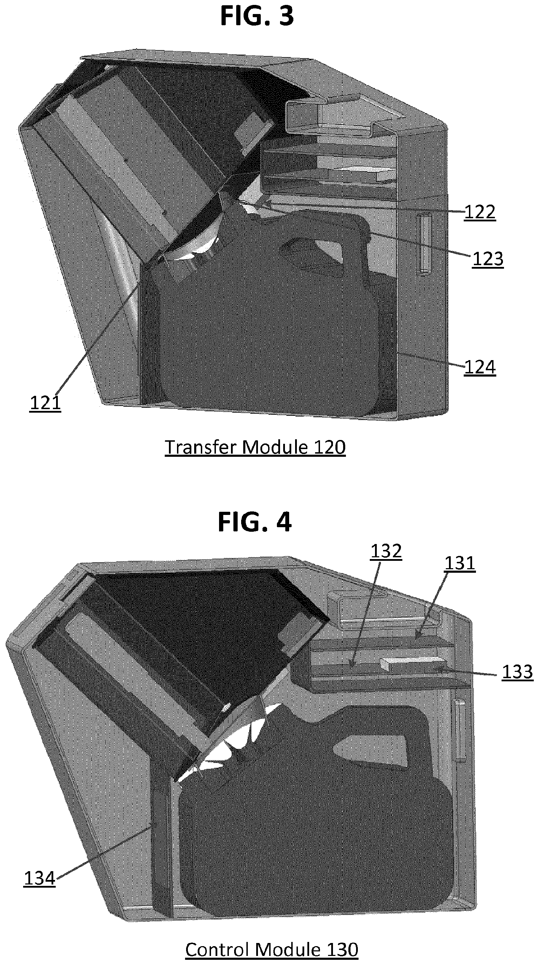

Figure 3 illustrates an example embodiment of a transfer module of the injection device disposal cabinet. In an embodiment, as depicted in the figure, the transfer module 120 includes an exit 121, an exit actuator 122, a disposal container interface 123, and a disposal container 124. In an embodiment, the exit 121 is implemented on a bottom end of the optical module 110. In an embodiment, the exit 121 is configured to hold the injection device 50 while the image acquisition device 114 captures an image of the injection device 50. In an embodiment, the exit 121 is a trapdoor. Further, in an embodiment the exit 121 may be electromechanically actuated (e.g., opened) by the exit actuator 122. In an embodiment, the exit 121 is opened without any additional user interaction. In an embodiment, once the exit 121 is opened, the injection device 50 is released into the disposal container 124. Specifically, the injection device 50 is released via the disposal container interface 123. In an embodiment, the disposal container interface 123 includes a conical shape. In an embodiment, the disposal container 124 (e.g., sharps disposal container) includes a plurality of safety features at its opening in order to limit the risk of puncture wounds associated with potentially contaminated injection devices 50. Further, in an embodiment, the opening of the disposal container is configured to be spread by the conically-shaped disposal container interface 123. As such, large injection devices will not be blocked by the safety features at the opening of the disposal container 124.

Further, in an embodiment, the transfer module 120 may include additional sensors to be implemented around the disposal container 124. In an embodiment, these additional sensors may be used to monitor the filling level of the disposal container 124 as well as the presence of the disposal container 124 in the disposal cabinet 100.

Figure 4 illustrates an example embodiment of a control module of the injection device disposal cabinet. As depicted in the figure, the control module 130 may include a first printed circuit board (PCB) 131, a second PCB 132, a battery 133, and a third PCB 134. In an embodiment, the first PCB 131 may be dedicated to data processing and radio frequency (RF) data transfer. Further, the first PCB may be placed on top of the disposal container 124 close to the image acquisition device 114. Further, in an embodiment, the second PCB 132 may be dedicated to power management and illumination control of the disposal cabinet 100. For example, in an embodiment, the second PCB 132 may be responsible for the monitoring and managing of the battery 133. In another embodiment, the first PCB 131 may also be dedicated to the power management and illumination control of the disposal cabinet 100. Further, in an embodiment, the third PCB 134 may dedicated to the filling level and container presence sensors discussed above.

The third PCB 134 may also host the driver for controlling the actuators for opening and closing the opening 103 and the exit 121. Further, the third PCB 134 may be located below the optical module 110 on the front side of the disposal container 124. Moreover, as a person of ordinary skill in the art would readily appreciate, it is possible that a single PCB could be utilized to perform all of the above functions (and more) described for PCB 131, 132 and 134.

In an embodiment, the identification of the injection device 50 can performed in the following steps. First, one of the user or the injection device 50 has to be detected by the proximity sensor associated with the activity area. Upon detection of one of the user or the injection device 50, the processor associated with the PCB 131 performs at least one of the following: (i) activates the disposal cabinet 100, (ii) opens the opening 103, and (iii) initiates the image acquisition device 114. Then, while the opening 103 is opened, the injection device 50 is discarded into disposal cabinet 100. Once the injection device 50 is secured in the optical module 110, the processor closes the opening and triggers the image acquisition device 114. The image acquisition device 114 then captures an image of the discarded injection device 50 while it is secured in the optical module 110 by the transparent holding elements 112 and the exit 121. Then, after the image of the injection device 50 is captured and stored in memory, the processor opens the exit 121 in order to release the injection device 50 into the disposal container 124. The processor then applies a series of detection filters (e.g., decision trees) on the stored image of the discarded injection device 50. After which, the processor performs a vote for each decision tree in order to select the most probable match among a database of injection device references (e.g., classes) stored locally in the disposal cabinet 100. Then, by adding the votes for all of the decision trees, the processor 131 finds the "best match" and, as such, identifies the discarded injection device 50. The identification result is then locally stored in memory. The processor may then automatically connect, via Bluetooth, to the user's smart device.

Further, in an embodiment, during the above-mentioned discarding process, the graphical user interface 102 may inform the user on the status of the status of (i) the system itself (e.g., on, processing, error, off), (ii) the filling level of the disposal container, (iii) the battery level, and (iv) the presence of a disposal container. Further, in an embodiment, the graphical user interface 102 may also provide wireless connection to an application located on the user's smart device and/or associated with another device or web application. Specifically, the disposal cabinet 100 may provide the results of the identification as well as the filling level of the disposal container to the user's smart device. The smart device can then further transfer this information to a cloud application. Further, in an embodiment, the cloud application can also be accessed by others (e.g., physicians and/or nurses) tending to the user. The disposal cabinet may then return to sleep mode.

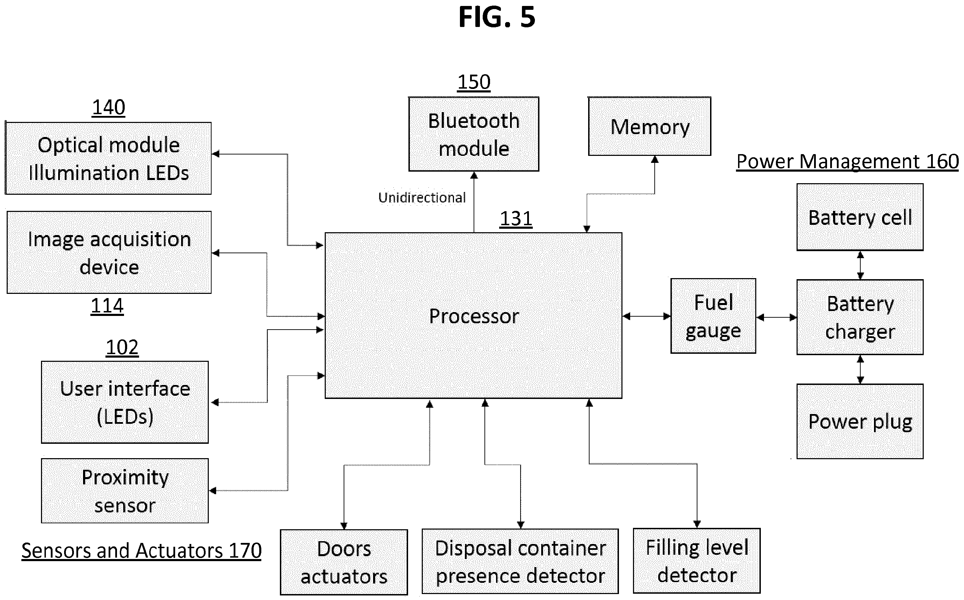

Figure 5 illustrates an example embodiment of the electronics architecture of the injection device disposal cabinet. In an embodiment, as depicted in the figure, the electronics architecture includes the image acquisition device 114, optical module illumination LEDs 140, Bluetooth module 150, power management 160, sensors and actuators 170, and user interface 102. In an embodiment, the image acquisition device 114 is a CMOS image sensor. In an embodiment, the CMOS image sensor may include an integrated image processor. Further, as regards to the optical module illumination LEDs 140, the disposal cabinet 100 may require a plurality of LEDs. Further, the Bluetooth module 150 permits the transfer of the data to the user's smart device or other devices. In an embodiment, the Bluetooth module may include a built-in antenna and a surface mount module that integrates the complete Bluetooth stack onboard. Further, in an embodiment, such a module is directly soldered on the main disposal cabinet PCB, i.e., PCB 131. Further, in an embodiment, to limit potential system intrusion, the disposal cabinet 100 may limit the communication with a set of unidirectional commands from the disposal cabinet 100 to the user's smartphone only. Further, as regards to the power management 160, the disposal cabinet 100 may include a battery cell, a battery charger circuit, a fuel gauge (which communicates with the processor 131) to monitor the battery charge status and capacity degradation allowing for an accurate prediction of the available capacity, a charge connector (e.g., power plug 107), and a main switch (e.g., power switch 106). Further, as regards to the sensors and actuators 170, the disposal cabinet 100 may include a proximity sensor, multiple door actuators (e.g., for the opening 103 and the exit 121, respectively), a disposal container presence detector, and a filling level detector. In an embodiment, the proximity sensor may be used to automatically activate the disposal cabinet 100 as an object (e.g., user and/or injection device 50) approaches the disposal cabinet 100. In an embodiment, the proximity sensor may include at least one of an infrared (IR) sensor, ultrasonic sensor, and a capacitive sensor. Further, the disposal container detector may include at least one of a mechanical switch and an IR sensor (e.g., reflective or transmissive). Further, in another embodiment, the disposal container detector may also be implemented with an available image sensor (e.g., image acquisition device 114). In an embodiment, the filling level detector may include at least one of an IR sensor, an ultrasonic sensor, and a mechanical finger (e.g., to sense the filling level). Further, in another embodiment, the filling level detector may also be implemented with an available image sensor (e.g., image acquisition device 114). In an embodiment, the multiple door actuators can be utilized to open and close the opening 103 and the exit 121, respectively. In an embodiment, the door actuators can be implemented with a low-cost servo or linear actuator in conjunction with an H-Bridge driver. Further, as depicted in the figure, the user interface 102 can be utilized to implement the following elements: status symbols (e.g., battery, filling level, pairing, etc.), LED backlight or OLED display, and a speaker.

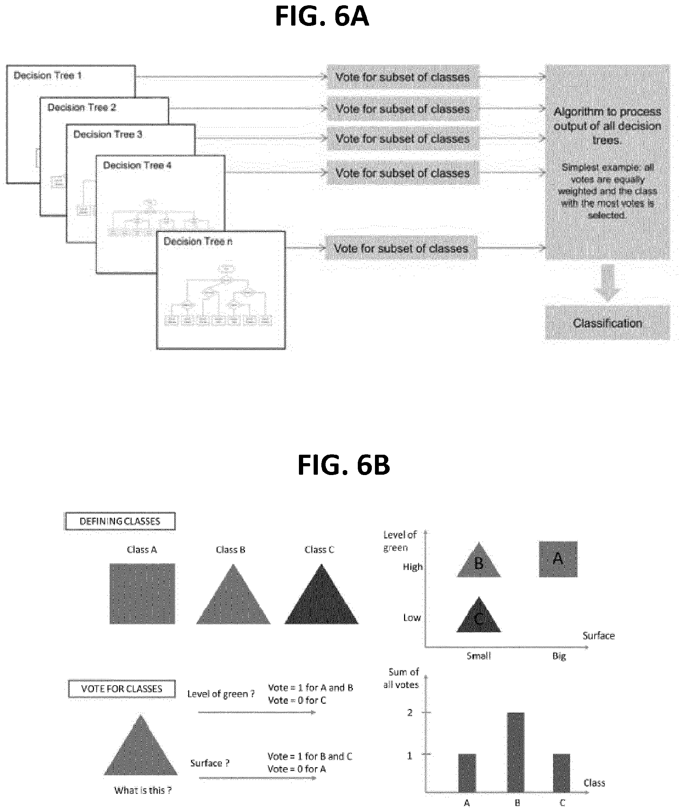

Figure 6A illustrates an example embodiment of the process utilized to identify an injection device. The identification algorithm is based on the concept of decision forests architecture (also called Random Forest). It consists of combining multiple uncorrelated decision trees with each of them giving votes for a subset of classes. As such, by combining the multiple decision trees, a more robust prediction of the class to which a sample belongs can be expected. A single decision tree (e.g., filter for certain discriminator) is not sufficient to identify a unique injection device 50; additional relevant decisions trees are required.

Figure 6B illustrates an example of the decision trees concept utilized in Figure 6A. This example is based on identifying a given picture among 3 possibilities (e.g., classes): a green square (class A), a green triangle (class B) and a red triangle (class C). Looking at only a single piece of information, like the amount of green color (the color decision tree), will not be sufficient to reliably identify the picture as belonging to only one of these classes. In fact, identifying the amount of green color alone will not be sufficient to distinguish between classes A and B. Further, the shape (or the surface) alone will also not be sufficient to discriminate between all references. Therefore, both decision trees have to be combined to get a reliable identification, which leads to the building of a decision forest. The identification relies on two steps, according to Figure 6B:
1. The first step consists of defining the attributes of classes that will be used (e.g., as a look-up table) during identification. Each decision tree is applied to each class (i.e., each separated reference image/product to identify) to get a "score." A table is then built containing the relevant class information for each tree. The look-up table includes the characteristics of each class for each attribute (e.g., the class for injection device A may have the following information; blue, conical, etc.). The look-up table is then stored in the relevant product (e.g., disposal cabinet 100).
2. The second step includes the voting of the classes, a step that takes place by selecting an image at random (e.g., with regard to the disposal cabinet 100, this happens each time a user throws a new syringe in). In order to identify which reference the image corresponds to, the two decision trees are applied and votes are attributed to the corresponding and predefined classes.
   Accordingly, applying the decision tree "Level of green color" gives a vote 1 for class A and B, since the picture to be identified contains a similar level of green as classes A and B, but gives a vote = 0 for class C since it is red. Further, applying the decision tree "Surface of the shape" gives a vote 1 for class B and C since the picture to be identified is a triangle, similar to classes B and C, but gives a vote = 0 to class A since it is a square. Therefore, by adding the votes obtained through each tree, the identification algorithm votes for the most probable class. Here, the picture to be identified obtains the largest score for class B, it is therefore identified as being a picture from class B.

With regard to the present invention, because of the wide variety of injection devices to be identified by the disposal cabinet 100, it is recommended that as many discriminators as possible are used to ensure good reliability.

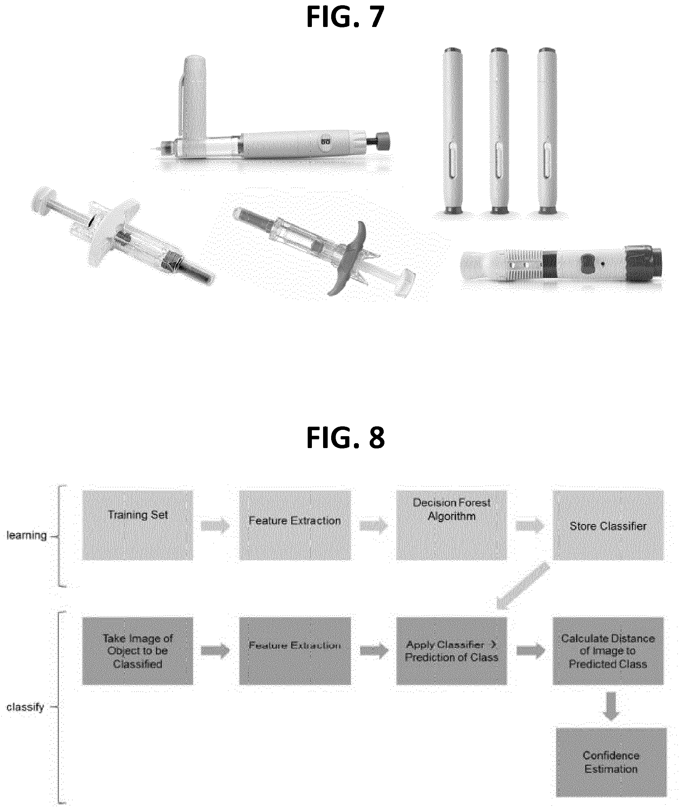

Figure 7 provides a non-exhaustive list of injection devices recognizable by the injection device disposal cabinet. As depicted in the figure, it can be seen that the injection devices share many similar aspects, and only minor discriminators can be found between the injection devices, which stresses the importance of finding relevant discriminators.

Figure 8 illustrates a flow diagram of the injection device identification process utilized in the disposal cabinet. As depicted in the figure, before the injection device can be identified, a training database is populated with a set of images of a variety of different injection devices. Further, to improve the recognition performance of the identification process, the training database can be populated with a set of characteristics for each class for each of the injection devices. For example, the training database can be populated with 92 images corresponding to 21 injection devices. Then, from this database, a plurality of features are extracted. For example, the features may be red, green, blue, yellow, cyan, magenta, total color pixels, length, width, surface, brightness (e.g., average value of all the pixel values within the acquired picture), contrast (e.g., sum of the pixels of the acquired picture after detecting discontinuities in brightness using the Sobel method), barcode (e.g., measurement of the profile of the light intensity measured along the injection device axis, helping to differentiate the various injection devices between them), and yellow-green content in a specified region of interest. Further, assuming the decision trees had been previously defined, the extracted features are then combined to generate bagged decision trees. Then, based on the generated decision trees, the look-up table (i.e., classifier) is generated. The look-up table is then stored for later use. Specifically, the look-up table is later utilized to classify (i.e., identify) any newly-discarded injection device. For example, after the disposal cabinet captures an image of the injection device (e.g., object), the processor (i) extracts the relevant features from the image and (ii) compares features with the look-up table to predict the class associated with the injection device. Then, in order to determine the confidence level of the prediction, a distance between the extracted feature and the value for this class in the look-up table is determined. This estimation is very useful to identify potential areas of improvement for the algorithm. For example if an injection device always has a medium score, a typical discriminator associated with it can be added in the features recognized by the decision forest. Accordingly, using the decision forest, combined with multiple discriminators, multiple injection devices can be recognized and differentiated from each other.

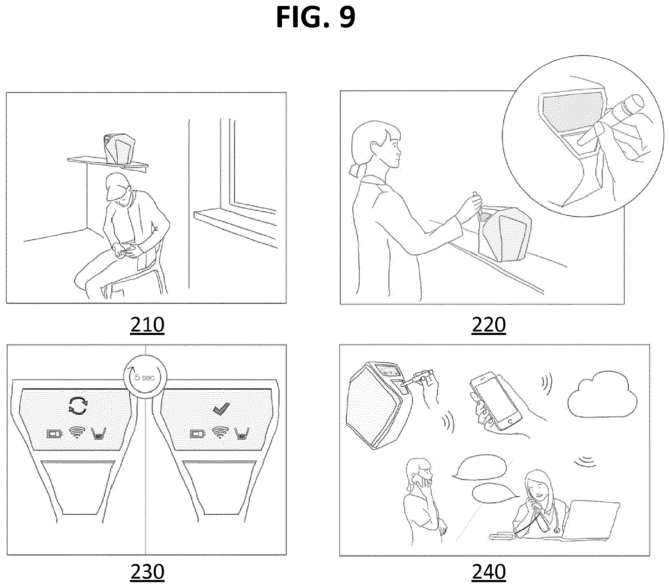

Figure 9 illustrates an example embodiment of a user interaction with disposal cabinet. For example, as depicted in step 210, the user performs an injection with a certain injection device 50. After the injection, the user may then discard the injection device 50 into the disposal cabinet 100 as depicted in step 220. Then, as depicted in step 230, the disposal cabinet 100 processes the injection device 50 and displays a confirmation to the user. In an embodiment, the processing may include: (i) capturing an image of the injection device 50, (ii) discarding the injection device 50 into the disposal container 124, and (iii) identifying the injection device 50. Further, in an embodiment, the processing may also include the monitoring of the filling level (e.g., of the injection devices 50) in the disposal container 124. Then, in step 240, the disposal cabinet 100 sends the injection device identification information to the user smart device application. In an embodiment, the disposal cabinet 100 may also send the filling level information to the user smart device application. In an embodiment, such information may also be sent to a cloud application to be accessed by other relevant parties, e.g., medical professionals. Accordingly, the medical professionals can also remotely track the user's treatment.

In the foregoing Description of Embodiments, various features may be grouped together in a single embodiment for purposes of streamlining the disclosure. This method of disclosure is not to be interpreted as reflecting an intention that the claims require more features than are expressly recited in each claim. Rather, as the following claims reflect, inventive aspects lie in less than all features of a single foregoing disclosed embodiment. Thus, the following claims are hereby incorporated into this Description of Embodiments, with each claim standing on its own as a separate embodiment of the invention.

Moreover, it will be apparent to those skilled in the art from consideration of the specification and practice of the present disclosure that various modifications and variations can be made to the disclosed systems without departing from the scope of the disclosure, as claimed. Thus, it is intended that the specification and examples be considered as exemplary only, with a true scope of the present disclosure being indicated by the following claims and their equivalents.

## Claims

1. A disposal cabinet for discarding and identifying a variety of injection devices, comprising:
an optical module, wherein the optical module includes an image acquisition device; and
a processor, wherein, upon detecting an object approaching the disposal cabinet, the processor is configured to:
open a first door in the optical module to receive an injection device,
capture, with the image acquisition device, an image of the injection device,
open a second door in the optical module to discard the received injection device into a disposal container coupled to the optical module, and
identify the injection device based on the captured image.

2. The disposal cabinet according to claim 1, wherein the injection devices is one of a syringe, cartridge, and auto-injector.

3. The disposal cabinet according to claim 1 or 2, wherein the optical module further includes a plurality of reflective surfaces and a plurality of transparent surfaces.

4. The disposal cabinet according to claim 3, wherein the plurality of reflective surfaces reflects images of the injection device to the image acquisition device.

5. The disposal cabinet according to claim 3 or 4, wherein the optical module includes two reflective surfaces beneath the injection device and another reflective surface above the injection device.

6. The disposal cabinet according to any one of claims 3 to 5, wherein each of the reflective surfaces is a mirror.

7. The disposal cabinet according to any one of claims 3 to 6, wherein the plurality of transparent surfaces holds the injection device as the image acquisition device captures the image of the injection device.

8. The disposal cabinet according to any one of the preceding claims, wherein the image acquisition device is one of a CMOS image sensor and a CCD image sensor.

9. The disposal cabinet according to any one of the preceding claims, further comprising:
a proximity sensor, wherein the proximity sensor detects the object approaching the disposal cabinet.

10. The disposal cabinet according to any one of the preceding claims, further comprising:
an electrically-actuated receiving door, wherein upon detecting the object approaching the disposal cabinet, the processor is configured to open the electrically-actuated receiving door, wherein the electrically-actuated receiving door is configured to receive the injection device.

11. The disposal cabinet according to any one of the preceding claims, further comprising:
an electrically-actuated discarding door, wherein after the image acquisition device captures the image of the injection device, the processor is configured to open the electrically-actuated discarding door, wherein the electrically-actuated discarding door discards the injection device into the disposal container coupled to the optical module.

12. The disposal cabinet according to any one of the preceding claims, wherein the identification of the injection device is transmitted via Bluetooth, to at least one of a smart device and a cloud application.

13. The disposal cabinet according to any one of the preceding claims, further comprising:
a disposal container detector, wherein the disposal container detector detects if the disposal container is located within the disposal cabinet.

14. The disposal cabinet according to any one of the preceding claims, further comprising:
a filling level detector, wherein the filling level detector detects a level of the injection devices in the disposal container.

15. The disposal cabinet of claim 14, wherein the level of the injection devices in the disposal container is transmitted, via Bluetooth, to at least one of a smartphone and a cloud application.

16. The disposal cabinet according to any one of the preceding claims, further comprising:
a user interface, wherein the user interface indicates the status of at least one cabin-related state.

17. The disposal cabinet of claim 16, wherein the at least one cabin-related state includes at least one of a battery-level of the disposal cabinet and a level of the injection devices in the disposal container.

18. The disposal cabinet according to any one of the preceding claims, wherein the identification of the injection device is performed with a plurality of decision trees.

19. The disposal cabinet according to any one of the preceding claims, wherein the identification of the injection device is performed based on at least one color and shape of the injection device.
